# EUROPEAN PATENT SPECIFICATION

(11) **EP 2 726 021 B1**
(45) Date of publication and mention of the grant of the patent: **04.11.2015**
(21) Application number: 12804095.3
(22) Date of filing: 27.06.2012
(51) Int. Cl.: A61F 2/38

(54) **POSTERIOR STABILIZED ORTHOPAEDIC PROSTHESIS ASSEMBLY**
ORTHOPÄDISCHE PROTHESENANORDNUNG MIT HINTERER STABILISIERUNG
ENSEMBLE PROTHÈSE ORTHOPÉDIQUE STABILISÉE POSTÉRIEURE

(30) Priority: 30.06.2011 US 201161503348 P
(43) Date of publication of application: 07.05.2014
(73) Proprietor: DePuy (Ireland), Ringaskiddy Cork (IE)
(72) Inventor: WYSS, Joseph G., Fort Wayne, Indiana 46814 (US); BENNETT, Travis D., Huntington, Indiana 46750 (US)
(74) Representative: Belcher, Simon James
(86) International application number: PCT/US2012/044356
(87) International publication number: WO 2013/003435

(56) References cited:
- WO-A1-2004/069104
- US-A- 3 840 905
- US-A1- 2009 326 665
- US-A1- 2009 326 666
- US-A1- 2009 326 666
- US-A1- 2010 016 979

## Description

The present invention relates generally to orthopaedic prostheses, and particularly to posterior stabilized orthopaedic prostheses for use in knee replacement surgery.

Joint arthroplasty is a well-known surgical procedure by which a diseased and/or damaged natural joint is replaced by a prosthetic joint. A typical knee prosthesis includes a tibial tray, a femoral component, and a polymer insert or bearing positioned between the tibial tray and the femoral component. A knee prosthesis is generally designed to duplicate the natural movement of the patient's joint. However, depending on the severity of the damage to the patient's joint, orthopaedic prostheses of varying mobility may be used. For example, in some patients, the posterior cruciate ligament maybe damaged, deficient, or removed during the orthopaedic surgical procedure. In such cases, a posterior stabilized knee orthopaedic prosthesis, which typically restricts or limits the posterior movement of the tibia relative to the femur, may be used.

US-A-2009/0326666 discloses an orthopaedic prosthesis having the features specified in the pre-characterising part of claim 1.

The invention provides an orthopaedic prosthesis assembly as defined in claim 1.

The first femoral component may be embodied as a primary femoral component and the second femoral component may be embodied as a secondary femoral component.

Optionally, the concave cam surface of the posterior cam surface of the first femoral component is concavely curved in the sagittal plane and the convex cam surface the posterior cam surface of the first femoral component is convexly curved in the sagittal plane. Additionally or alternatively, the concave cam surface and the convex cam surface of the posterior cam surface of the first femoral component may be concavely curved in the medial-lateral direction. Optionally, the posterior cam surface of the second femoral component is concavely curved in the medial-lateral direction. Additionally, in some constructions, the posterior cam surfaces of the first and second femoral components are each concavely curved in the medial-lateral direction.

Additionally, in some constructions, the convex cam surface of the spine of the tibial bearing may be convexly curved in the sagittal plane and the concave cam surface of the spine is concavely curved in the sagittal plane. Additionally or alternatively, the concave cam surface and the convex cam surface of the spine may be convexly curved in the transverse plane. Optionally, the radius of curvature in the transverse plane of the concave cam surface of the spine may be substantially equal to the radius of curvature in the transverse plane of the convex cam surface of the spine. Optionally, the convex cam surface of the spine of the tibial bearing maybe located superiorly relative to the concave cam surface of the spine.

Optionally, the degrees of flexion of the first range of flexion may be less than the degrees of flexion of the second range of flexion. Additionally, in some constructions, the concave cam surface of the spine of the tibial bearing may be defined by a first radius of curvature and the convex cam surface of the spine may be defined by a second radius of curvature that is different from the first radius of curvature. Additionally or alternatively, concave cam surface of the posterior cam surface of the first femoral component may be defined by a third radius of curvature and the convex cam surface of the posterior cam surface of the first femoral component may be defined by a fourth radius of curvature, the third radius of curvature being different from the fourth radius of curvature.

An orthopaedic prosthesis assembly may include a tibial bearing, a primary femoral component and a revision femoral component. The tibial bearing may include a platform and a spine extending upwardly from the platform. The spine may include a posterior cam surface having a substantially "S"-shaped cross-section in the sagittal plane.

The primary femoral component may be configured to be coupled to a surgically-prepared distal end of a femur and include a posterior cam having a posterior cam surface. The posterior cam surface may have a substantially "S"-shaped cross-section in the sagittal plane. The revision femoral component may be configured to be coupled to the surgically-prepared distal end of the femur and include a posterior cam having a posterior cam surface that is convexly curved in the sagittal plane. Each of the primary and revision femoral components may be configured to couple to the tibial bearing and articulate with the tibial bearing such that the posterior cam surface of the respective primary and revision femoral component articulates on the posterior cam surface of the spine of the tibial bearing during a range of flexion.

Optionally, the posterior cam surfaces of the primary and the revision femoral components are each concavely curved in the transverse plane. Additionally, the posterior cam surface of the tibial bearing may include a concave cam surface and a convex cam surface in the sagittal plane. The concave cam surface may be concavely curved in the sagittal plane and the convex cam surface may be convexly curved in the sagittal plane. Additionally, in some constructions, the concave cam surface and the convex cam surface of the posterior cam surface of the tibial bearing are curved in the transverse plane.

Optionally, the posterior cam surface of posterior cam of the primary femoral component may include a concave cam surface and a convex cam surface. The concave cam surface may be concavely curved in the sagittal plane and the convex cam surface may be convexly curved in the sagittal plane. Additionally, in some constructions, each of the posterior cams of the primary and the revision femoral components may be configured to rotate about the spine of the tibial bearing in the transverse plane when the respective primary and revision femoral component articulates with the tibial bearing.

A posterior stabilized knee orthopaedic prosthesis assembly may include a tibial bearing, a primary femoral component, and a revision femoral component. Each of the primary and revision femoral components may be configured to separately couple with the tibial bearing and articulate on the tibial bearing during a range of flexion. The tibial bearing may include a platform having a medial bearing surface and a lateral bearing surface and a spine extending upwardly from the platform between the medial bearing surface and the lateral bearing surface. The spine may include a posterior side having a superior cam surface and an inferior cam surface. The superior cam surface may be convexly curved in the sagittal plane and the inferior cam surface may be concavely curved in the sagittal plane. The superior cam surface and the inferior cam surface may be convexly curved in the transverse plane.

The primary femoral component may include a primary lateral condyle configured to articulate with the lateral bearing surface of the tibial bearing, a primary medial condyle configured to articulate with the medial bearing surface, and a primary posterior cam positioned in a primary intercondylar notch defined between the primary lateral condyle and the primary medial condyle. The primary posterior cam may include a primary concave cam surface and a primary convex cam surface. The primary concave posterior cam surface may be positioned to initially contact the superior cam surface of the spine at a first degree of flexion. Additionally, the primary convex cam surface may be positioned to initially contact the inferior cam surface of the spine at a second degree of flexion greater than the first degree of flexion.

The revision femoral component may include a revision lateral condyle configured to articulate with the lateral bearing surface of the tibial bearing, a revision medial condyle configured to articulate with the medial bearing surface, and a revision posterior cam positioned in a revision intercondylar notch defined between the revision lateral condyle and the revision medial condyle. The revision posterior cam may include a revision convex cam surface that is positioned to initially contact the superior cam surface of the spine at a third degree of flexion and initially contact the inferior cam surface of the spine at a fourth degree of flexion greater than the third degree of flexion.

The detailed description particularly refers to the following figures, in which:
FIG. 1 is an exploded perspective view of an orthopaedic prosthesis assembly.
FIG. 2 is a cross-sectional view of a tibial bearing of the orthopaedic prosthesis assembly of FIG. 1.
FIG. 3 is a cross-sectional view of another tibial bearing of the orthopaedic prosthesis assembly of FIG. 1.
FIG. 4 is a cross-sectional view of a primary femoral component of the orthopaedic prosthesis assembly of FIG. 1.
FIG. 5 is a cross-sectional view of another primary femoral component of the orthopaedic prosthesis assembly of FIG. 1.
FIG. 6 is a cross-sectional view of a revision femoral component of the orthopaedic prosthesis assembly of FIG. 1.
FIGS. 7 to 10 are side elevational views of the orthopaedic prosthesis of FIG. 1 using the primary femoral component of FIG. 4 at various degrees of flexion.
FIGS. 11 to 14 are side elevational views of the orthopaedic prosthesis of FIG. 1 using the revision femoral component of FIG. 6 at various degrees of flexion.
FIG. 15 is a top plan view of another tibial bearing of the orthopaedic prosthesis of FIG. 1.
FIG. 16 is a cross-sectional plan view of the tibial bearing of FIG. 15 having a portion of the spine removed.
FIG. 17 is a side elevational view of the orthopaedic prosthesis assembly 10 using the primary femoral component of FIG. 4 and the tibial bearing of FIGS. 15 and 16 positioned in an early degree of flexion.
FIG. 18 is a cross-sectional view of the orthopaedic prosthesis assembly of FIG. 17 taken generally along the section line 18-18.
FIG. 19 is a side elevational view of the orthopaedic prosthesis assembly 10 of FIG. 17 positioned in a late degree of flexion.
FIG. 20 is a cross-sectional view of the orthopaedic prosthesis of FIG. 7 taken generally along the section line 19-19.

Terms representing anatomical references, such as anterior, posterior, medial, lateral, superior and inferior, may be used throughout this document to refer to both the orthopaedic implants described herein and a patient's natural anatomy. Such terms have well-understood meanings in both the study of anatomy and the field of orthopaedics. Use of such anatomical reference terms in this document is intended to be consistent with their well-understood meanings unless noted otherwise.

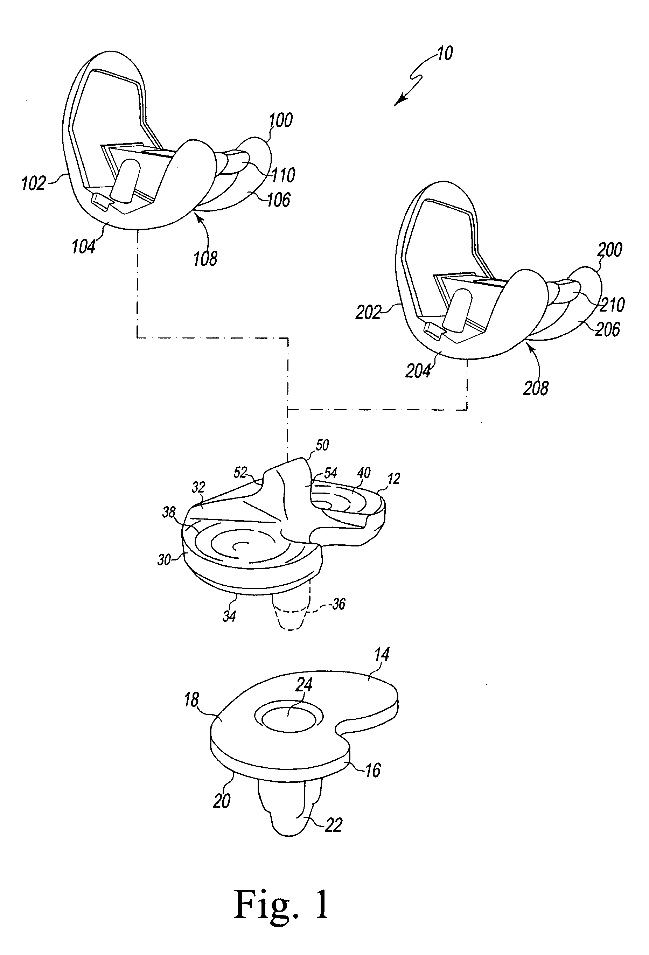

Referring now to FIG. 1, a posterior stabilized knee orthopaedic prosthesis assembly 10 includes a tibial insert or bearing 12, a tibial tray 14, a primary femoral component 100, and a revision femoral component 200. The primary and revision femoral components 100, 200 are each configured to separately couple to and articulate with the tibial bearing 12 during use. That is, based on the particular orthopaedic surgery to be performed, the preference of the orthopaedic surgeon, and/or other factors, the surgeon may select one of the femoral components 100, 200 to use with the tibial bearing 12 in the orthopaedic surgical procedure. Typically, the primary femoral component 100 is used for the initial orthopaedic surgical procedure on the patient (e.g., the first total knee arthroplasty procedure performed on a patient's particular knee), and the revision femoral component 200 is sued for subsequent orthopaedic surgical procedures (e.g., procedures to correct misalignment, loosening of components, etc.). An orthopaedic surgeon may use either of the femoral components 100, 200 during any particular orthopaedic surgery. However, because each of the primary femoral component 100 and the revision femoral component 200 is configured to be used with the same tibial bearing 12, the overall number of components of a typical primary/revision orthopaedic prosthesis assembly is reduced because a single tibial bearing 12 is used with either femoral components 100, 200.

The tibial bearing 12 is illustratively formed from a polymer material such as a ultra-high molecular weight polyethylene (UHMWPE), but may be formed from other materials, such as a ceramic material, a metallic material, a bio-engineered material, or the like. The illustrative tibial bearing 12 is embodied as a rotating or mobile tibial bearing and is configured to rotate relative to the tibial tray 14 during use. However, the tibial bearing 12 may be embodied as a fixed tibial bearing, which may be limited or restricted from rotating relative the tibial tray 14.

The tibial tray 14 is configured to be secured to a surgically-prepared proximal end of a patient's tibia (not shown). The tibial tray 14 may be secured to the patient's tibia via use of bone adhesive or other attachment means. The tibial tray 14 includes a platform 16 having a top surface 18 and a bottom surface 20. Illustratively, the top surface 18 is generally planar and may be highly polished. The tibial tray 14 also includes a stem 22 extending downwardly from the bottom surface 20 of the platform 16. A cavity or bore 24 is defined in the top surface 18 of the platform 16 and extends downwardly into the stem 22. The bore 24 is formed to receive a complimentary stem of the tibial bearing 12 as discussed below.

As discussed above, the tibial bearing 12 is configured to be coupled with the tibial tray 14. The tibial bearing 12 includes a platform 30 having an upper bearing surface 32 and a bottom surface 34. In the construction shown in the drawings, in which the tibial bearing 12 is embodied as a rotating or mobile tibial bearing, the bearing 12 includes a stem 36 extending downwardly from the bottom surface 34 of the platform 30. When the tibial bearing 12 is coupled to the tibial tray 14, the stem 36 is received in the bore 24 of the tibial tray 14. In use, the tibial bearing 12 is configured to rotate about an axis defined by the stem 36 relative to the tibial tray 14. In constructions in which the tibial bearing 12 is embodied as a fixed tibial bearing, the bearing 12 may or may not include the stem 36 and/or may include other devices or features to secure the tibial bearing 12 to the tibial tray 14 in a non-rotating configuration.

The upper bearing surface 32 of the tibial bearing 12 includes a medial bearing surface 38, a lateral bearing surface 40, and a spine 50 extending upwardly from the platform 30. The medial and lateral bearing surfaces 38, 40 are configured to receive or otherwise contact corresponding medial and lateral condyles of one of the femoral components 100, 200 as discussed in more detail below. As such, the bearing surfaces 38, 40 may have concave contours. The spine 50 is positioned between the bearing surfaces 38, 40 and includes an anterior side 52 and a posterior side 54.

Each of the primary femoral component 100 and the revision femoral component 200 is illustratively formed from a metallic material such as cobalt-chromium or titanium, but may be formed from other materials, such as a ceramic material, a polymer material, a bio-engineered material, or the like. As discussed above, each of the femoral components 100, 200 is configured to articulate with the tibial bearing 12 and has similar geometry to each other.

The primary femoral component 100 is configured to be coupled to a surgically-prepared surface of the distal end of a patient's femur (not shown). The femoral component 100 maybe secured to the patient's femur via use of bone adhesive or other attachment means. The femoral component 100 includes an articulating surface 102 having a pair of spaced apart medial and lateral condyles 104, 106. In use, the condyles 104, 106 replace the natural condyles of the patient's femur and are configured to articulate on the corresponding bearing surfaces 38, 40 of the platform 30 of the tibial bearing 12.

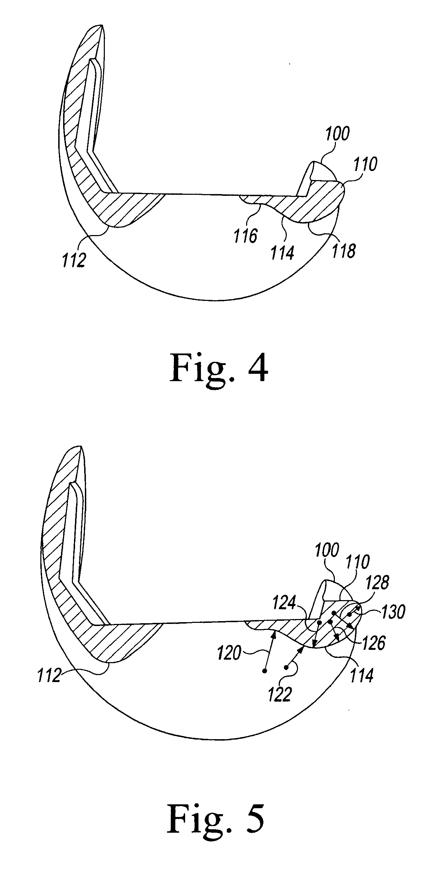

The condyles 104, 106 are spaced apart to define an intercondylar notch or recess 108 between them. A posterior cam 110 and an anterior cam 112 (see FIG. 4) are positioned in the intercondylar notch 108. The posterior cam 110 is located toward the posterior side of the femoral component 100 and is configured to engage or otherwise contact the spine 50 of the tibial bearing 12 during flexion as described below.

The revision femoral component 200 is similar to the primary femoral component 100 and is also configured to be coupled to a surgically-prepared surface of the distal end of a patient's femur (not shown) instead of the primary femoral component 100. As with the primary femoral component 100, the femoral component 200 may be secured to the patient's femur using bone adhesive or other attachment means. The femoral component 200 includes an articulating surface 202 having a pair of spaced apart medial and lateral condyles 204, 206. In use, the condyles 204, 206 replace the natural condyles of the patient's femur and are configured to articulate on the corresponding bearing surfaces 38, 40 of the platform 30 of the tibial bearing 12.

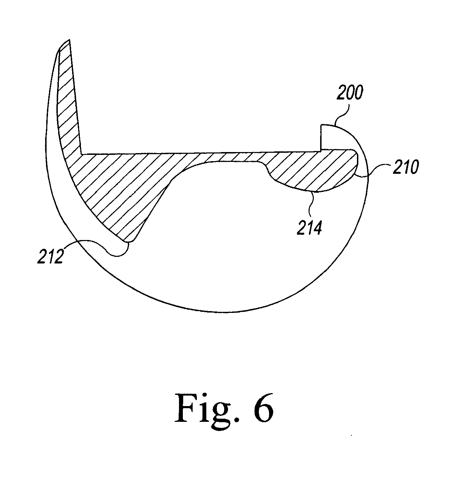

The condyles 204, 206 are spaced apart to define an intercondylar notch or recess 208 between them. A posterior cam 210 and an anterior cam 212 (see FIG. 6) are positioned in the intercondylar notch 208. The posterior cam 210 is located toward the posterior side of the femoral component 200 and is configured to engage or otherwise contact the spine 50 of the tibial bearing 12 during flexion as described below.

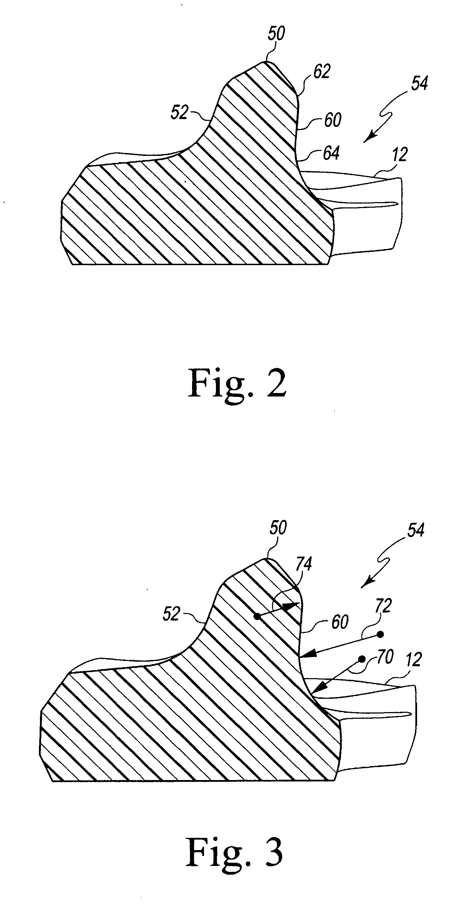

Referring now to FIGS. 2 and 3, the spine 50 of the tibial bearing 12 includes a cam surface 60 on the posterior side 54 of the spine 50. The cam surface 60 is configured to contact and articulate with the posterior cams 110, 210 of the femoral components 100, 200 during use. Illustratively, the cam surface 60 of the spine 50 has a substantially "S"-shaped cross-sectional profile in the sagittal plane. In particular, the cam surface 60 includes a convex cam surface 62 and a concave cam surface 64. In the construction shown in the drawings, the convex cam surface 62 is positioned superiorly relative to the concave cam surface 64. The cam surfaces 62, 64 of the spine 50 may have similar or different radius of curvatures. For example, the concave cam surface 64 may have a radius of curvature substantially larger than the radius of curvature of the convex cam surface 62. However, the concave cam surface 64 may have a radius of curvature that is substantially equal to or less than the radius of curvature of the convex cam surface 62.

In some constructions, the curvature of the cam surfaces 62, 64 may be defined by a single radius of curvature. The particular radius of curvature of the cam surfaces 62, 64 (i.e., the "size" of the cam surfaces) maybe dependent upon a number of criteria such as the size of the implant, the shape or geometry of the articulating surface of the posterior cams 110, 210 of the femoral components 100, 200, and/or the like. However, the convex cam surface 62 and the concave cam surface 64 of the tibial bearing 12 may be formed from multiple radii of curvature. For example, in the construction shown in FIG. 3, the concave cam surface 64 is defined by a radius of curvature 70 and a radius of curvature 72, each of which is tangent to the other. In one particular construction, the radius of curvature 70 is about 9.00 mm and the radius of curvature 72 is about 13.00 mm. The convex cam surface 62 is defined by a radius of curvature 74. In one particular construction, the radius of curvature 74 is about 8.00 mm. Of course, a larger or lesser number of radii of curvature may be used define the cam surfaces 62, 64. Additionally, the radii of curvature 70, 72, 74 may have other values.

Referring now to FIGS. 4 and 5, the posterior cam 110 of the primary femoral component 100 includes a cam surface 114 configured to contact the cam surface 60 of the spine 50 during use. Similar to the cam surface 60 of the spine 50, the cam surface 114 of the posterior cam 110 has a substantially "S"-shaped cross-sectional profile in the sagittal plane. In particular, the cam surface 114 includes a concave cam surface 116 and a convex cam surface 118. In the construction shown in the drawings, the convex cam surface 118 is positioned posteriorly to the concave cam surface 116. The cam surfaces 116, 118 may have similar or different radius of curvatures. For example, the convex cam surface 118 may have a radius of curvature substantially larger than the radius of curvature of the concave cam surface 116. However, the convex cam surface 118 may have a radius of curvature that is substantially equal to or less than the radius of curvature of the concave cam surface 116.

Optionally, the curvature of the cam surfaces 116, 118 may be defined by a single radius of curvature. The particular radius of curvature of the cam surfaces 116, 118 (i.e., the "size" of the cam surfaces) may be dependent upon a number of criteria such as the size of the implant, the shape or geometry of the cam surface 60 of the spine 50 of the tibial bearing 12, and/or the like. However, the concave cam surface 116 and the convex cam surface 118 of the femoral component 100 may be formed from multiple radii of curvature. For example, in the construction shown in FIG. 5, the concave cam surface 116 is defined by a radius of curvature 120 and a radius of curvature 122, each of which is tangent to the other. In one particular construction, the radius of curvature 120 is about 10.42 mm and the radius of curvature 122 is about 8.13 mm. Additionally, the convex cam surface 118 is defined by a plurality of radii of curvature 124, 126, 128, and 130. Each of the radii of curvature 124, 126, 128, 130 is tangent with the each adjacent radius of curvature. In one particular construction, the radius of curvature 124 is about 7.14 mm, the radius of curvature 126 is about 7.01 mm, the radius of curvature 128 is about 7.30 mm, and the radius of curvature 130 is about 2.30 mm. A larger or lesser number of radii of curvature may be used define the cam surfaces 116, 118. Additionally, the radii of curvature 124, 126, 128, 130 may have other values.

Referring now to FIG. 6, similar to the posterior cam 110 of the primary femoral component 100, the posterior cam 210 of the revision femoral component 200 includes a cam surface 214 configured to contact the cam surface 60 of the spine 50 during use. However, unlike the posterior cam 110 of the primary femoral component 100, the cam surface 214 does not have a substantially "S"-shaped cross-sectional profile in the sagittal plane. Rather, the cam surface 214 is embodied as a substantially cam surface. In the construction shown in the drawings, the cam surface 214 is uniformly convex. That is, the cam surface 214 does not include a concave section. In one construction, the convex cam surface 214 is defined by a single radius of curvature. However, the convex cam surface 214 may be defined by multiple radii of curvature, each having a different length so as to define a convex cam surface having multiple convex curved surface sections that cooperate to define the convex cam surface 214.

In use, an orthopaedic surgeon may use either the primary femoral component or the revision femoral component depending on the patient's anatomy, the type of orthopaedic surgical procedure being performed, the surgeon's preference, and/or other criteria. As discussed above, each of the femoral components 100, 200 is configured to articulate with the tibial bearing 12. During flexion, the posterior cams 110, 210 of the femoral components 100, 200 are configured to contact the spine 50 of the tibial bearing 12.

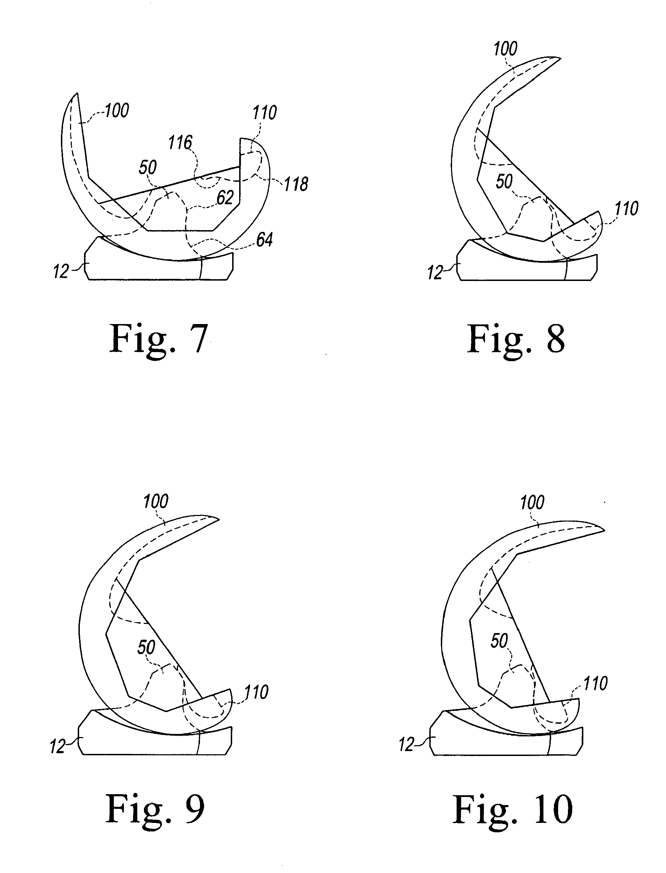

In constructions in which the primary femoral component 100 is used, the concave cam surface 116 of the posterior cam 110 contacts the convex cam surface 62 of the spine 50 during early flexion. As flexion of the femoral component 100 and tibial bearing 12 is increased, the contact between the posterior cam 110 and the spine 50 transitions from contact between the concave cam surface 116 of the posterior cam 110 and the convex cam surface 62 of the spine 50 to contact between the convex cam surface 118 of the posterior cam 110 and the concave cam surface 64 of the spine 50 during late flexion. For example, as shown in FIG. 7, when the femoral component 100 and tibial bearing 12 are in extension or are otherwise not in flexion (e.g., a flexion of about 0 degrees), the posterior cam 110 is not in contact with the spine 50. However, during early flexion as shown in FIG 8, the posterior cam 110 of the femoral component 100 contacts the spine 50 of the tibial bearing 12. As the femoral component 100 and tibial bearing 12 are moved in flexion, the concave cam surface 116 of the posterior cam 110 initially contacts the convex cam surface 62 of the spine 50 at a predetermined degree of flexion and maintains contact through early flexion. In the construction shown in the drawings, the femoral component 100 and the tibial bearing 12 are configured such that the cam surfaces 116, 62 initially contact each other at about 60 degrees of flexion. However, the degree of flexion at which initial contact between the posterior cam 110 and the spine 50 is established may be determined based on particular criteria such as the size of the orthopaedic prosthesis 10, the shape or geometry of the articulating surface of the primary femoral component 100 and/or the tibial bearing 12, and/or the like.

After early flexion, the contact between the posterior cam 110 and the spine 50 transitions from the cam surfaces 116, 62 to the cam surfaces 118, 64. For example, as shown in FIG. 9, the contact between the posterior cam 110 and the spine 50 begins transitioning to the cam surfaces 118, 64 at about 80 degrees. At this degree of flexion, initial contact between the convex cam surface 118 of the posterior cam 110 and the concave cam surface 64 of the spine 50 may be established. During late flexion of the femoral component 100 and tibial bearing 12, the convex cam surface 118 maintains contact with the concave cam surface 64 as shown in FIG. 10.

Contact between the posterior cam 110 and the spine 50 is maintained throughout the range of early and late flexion. The particular range of early flexion (i.e., the range at which the concave cam surface 116 of the posterior cam 110 contacts the convex cam surface 62 of the spine 50) and late flexion (i.e., the range at which the convex cam surface 118 of the posterior cam 110 contacts the concave cam surface 64 of the spine 50) of the femoral component 100 and the tibial bearing 12 may be dependent upon one or more criteria such as the size of the primary femoral component 100 and the tibial bearing 12, the shape or geometry of the articulating cam surfaces of the tibial bearing 12 and the primary femoral component 100, or the like. In the construction shown in the drawings, the primary femoral component 100 and the tibial bearing 12 are configured to have an early flexion range of about 50 degrees to about 80 degrees and a late flexion range of about 80 degrees to about 150 degrees, but other ranges of flexion may be used. The range of early and late flexion is determined, in part, based on the radius of curvature of the cam surfaces 116, 118, 62, 64. As such, the range of early and late flexion of the interaction between the primary femoral component 100 and the tibial bearing 12 may be configured by adjusting the radius of curvature of the cam surfaces 116, 118, 62, 64.

Because the cam surface 114 of the posterior cam 110 includes the concave cam surface 116 and the convex cam surface 118 and the cam surface 54 of the spine 50 includes the convex cam surface 62 and the concave cam surface 64, the contact surface area between the posterior cam 110 of the primary femoral component 100 and the spine 50 is increased through the flexion range relative to orthopaedic prostheses in which the posterior cam and/or the spine include planar cam surfaces or cam surfaces having only a concave or convex surface. For example, the contact area between the posterior cam 110 and the spine 50 is increased in early flexion due to the interface between the concave cam surface 116 of the posterior cam 110 and the convex cam surface 62 of the spine 50. Additionally, in late flexion, the contact area between the posterior cam 110 and the spine 50 is increased in later degrees of flexion due to the interface between the convex cam surface 118 of the posterior cam 110 and the concave cam surface 64 of the spine 50.

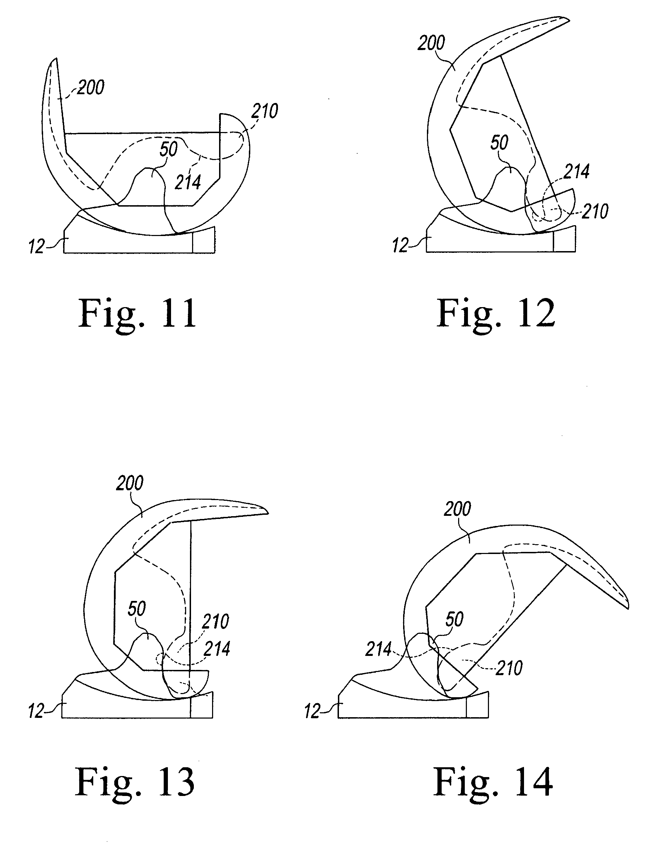

Referring now to FIGS. 11 to 14, when the revision femoral component 200 is used, the cam surface 214 of the posterior cam 210 contacts the convex cam surface 62 of the spine 50 of the tibial bearing 12 during early flexion. As discussed above, the cam surface 214 of the femoral component 200 is substantially uniformly convex in the sagittal plane relative to the cam surface 114 of the femoral component 200, which includes the concave cam surface 116 and the convex cam surface 118. As flexion of the femoral component 200 on the tibial bearing 12 is increased, the contact between the posterior cam 210 and the spine 50 transitions from contact between the convex cam surface 214 of the posterior cam 210 and the convex cam surface 62 of the spine 50 to contact between the convex cam surface 214 of the posterior cam 210 and the concave cam surface 64 of the spine 50 during late flexion. For example, as shown in FIG. 11, when the femoral component 200 and tibial bearing 12 are in extension or are otherwise not in flexion (e.g., a flexion of about 0 degrees), the posterior cam 210 is not in contact with the spine 50. However, during early flexion as shown in FIG 12, the posterior cam 210 of the femoral component 200 contacts the spine 50 of the tibial bearing 12. As the femoral component 200 and tibial bearing 12 are moved in flexion, the posterior cam 210 initially contacts the convex cam surface 62 of the spine 50 at a predetermined degree of flexion and maintains contact through early flexion. In the construction shown in the drawings, the revision femoral component 200 and the tibial bearing 12 are configured such that the cam surfaces 214, 62 initially contact each other at about 60 degrees of flexion. However, the degree of flexion at which initial contact between the posterior cam 210 and the spine 50 is established may be determined based on particular criteria such as the size of the orthopaedic prosthesis 10, the shape or geometry of the articulating surface of the revision femoral component 200 and/or the tibial bearing 12, and/or the like.

After early flexion, the contact between the posterior cam 210 and the spine 50 transitions from the cam surfaces 210, 62 to the cam surfaces 214, 64. For example, as shown in FIG. 13, the contact between the posterior cam 210 and the spine 50 begins transitioning to the cam surfaces 214, 64 at about 80 degrees. At this degree of flexion, initial contact between the convex cam surface 214 of the posterior cam 210 and the concave cam surface 64 of the spine 50 may be established. During late flexion of the revision femoral component 200 and the tibial bearing 12, the convex cam surface 214 maintains contact with the concave cam surface 64 of the spine 50 as shown in FIG. 14

As with the posterior cam 110, contact between the posterior cam 210 and the spine 50 is maintained throughout the range of early and late flexion. The particular range of early flexion (i.e., the range at which the convex cam surface 214 of the posterior cam 210 contacts the convex cam surface 62 of the spine 50) and late flexion (i.e., the range at which the convex cam surface 214 of the posterior cam 210 contacts the concave cam surface 64 of the spine 50) of the revision femoral component 200 and the tibial bearing 12 may be dependent upon one or more criteria such as the size of the revision femoral component 200 and the tibial bearing 12, the shape or geometry of the articulating cam surfaces of the tibial bearing 12 and the revision femoral component 200, or the like. In the construction shown in the drawings, the revision femoral component 200 and the tibial bearing 12 are configured to have an early flexion range of about 50 degrees to about 80 degrees and a late flexion range of about 80 degrees to about 150 degrees, but other ranges of flexion may be used. The range of early and late flexion is determined, in part, based on the radius of curvature of the cam surfaces 214, 62, 64. As such, the range of early and late flexion of the interaction between the revision femoral component 200 and the tibial bearing 12 may be configured by adjusting the radius of curvature of the cam surfaces 214, 62, 64.

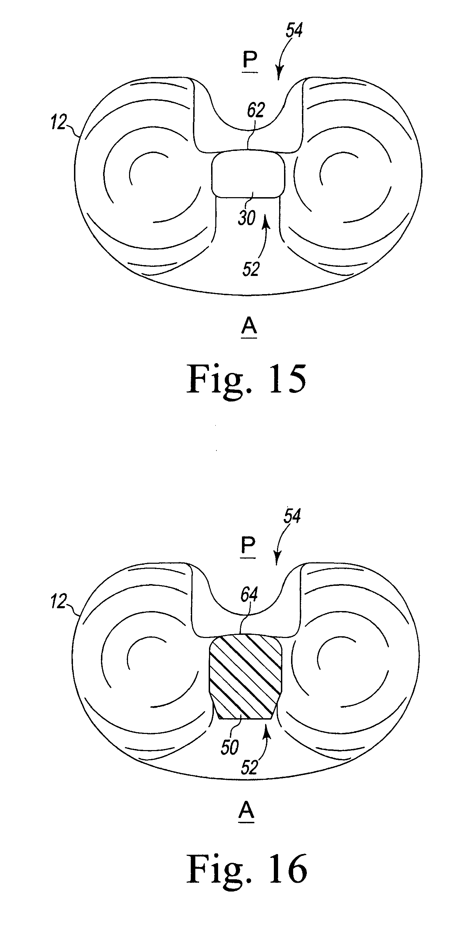

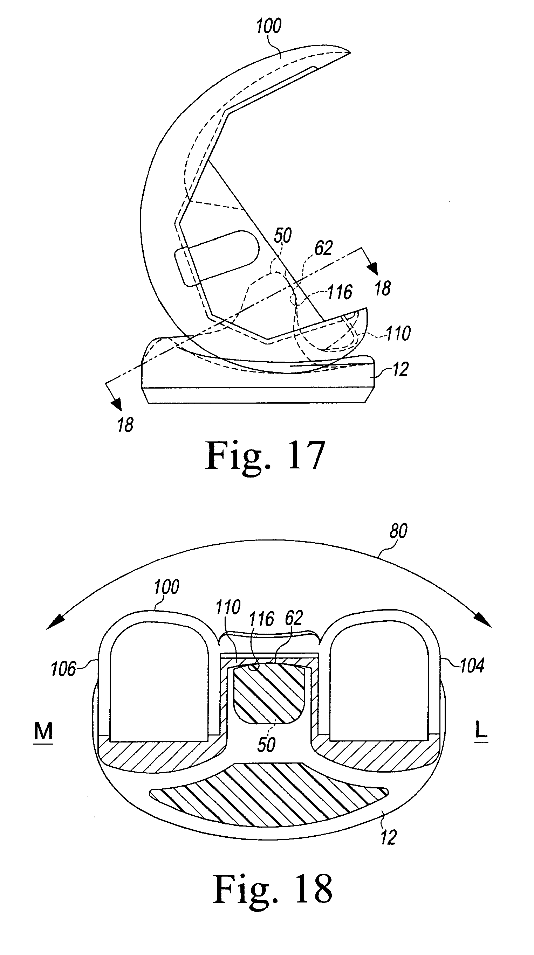

Referring now to FIGS. 15 and 17, the posterior side 54 of the spine 50 may also be curved in the transverse plane. That is, each of the superior, convex cam surface 62 and the inferior, concave cam surface 64 may be convex in the transverse plane direction. For example, as shown in FIG. 15, the convex cam surface 62 of the spine 50 maybe convexly curved in the transverse plane. Additionally, as shown in FIG. 16, the concave cam surface 64 of the spine 50 may be convexly curved in the transverse plane. The radius of curvature in the transverse plane of the convex cam surface 62 and the concave cam surface 64 may be substantially equal or different. For example, the radius of curvature in the transverse plane of the concave cam surface 64 may be greater than the radius of curvature in the transverse plane of the convex cam surface 62. Alternatively, the radius of curvature in the transverse plane of the convex cam surface 62 may be greater than the radius of curvature in the transverse plane of the concave cam surface 64.

When the cam surfaces 62, 64 of the spine 50 are curved in the transverse plane, the posterior cams 110, 210 of the femoral components 100, 200 articulate on the cam surfaces 62, 64 in the transverse plane such that the femoral components 100, 200 rotate an amount about the spine 50. An example of such rotation using the primary femoral component 100 and the tibial bearing 12 is shown in FIGS. 17 to 20. For example, as shown in FIGS. 17 and 18, when the concave cam surface 116 of the posterior cam 110 of the femoral component 100 is in contact with the convex cam surface 62 of the spine 50 during early flexion, the femoral component 100 may rotate about the spine 50 in a generally medial-lateral direction in the transverse plane as indicated by arrow 80. In such constructions, the concave cam surface 116 of the posterior cam 110 may be substantially planar in the medial-lateral direction. Alternatively, similar to the convex cam surface 62 of the spine 50, the concave cam surface 116 of the posterior cam 110 of the femoral component 100 may also be curved in the medial-lateral direction. For example, as shown in FIG. 18, the concave cam surface 116 may be concavely curved in the medial-lateral direction. In some constructions, the radius of curvature in the medial-lateral direction of the concave cam surface 116 may be substantially equal to the radius of curvature in the transverse plane of the convex cam surface 62 of the spine 50. Alternatively, the radius of curvature in the medial-lateral direction of the concave cam surface 116 may be greater or less than the radius of curvature in the transverse plane of the convex cam surface 62. The amount of rotation between the femoral component 100 and the tibial bearing 12 during early flexion may be adjusted based on the radius of curvatures in the transverse plane of the cam surfaces 116, 62. For example, an increased amount of rotation during early flexion of the orthopaedic prosthesis may be obtained by decreasing the radius of curvature in the transverse plane of the convex cam surface 62.

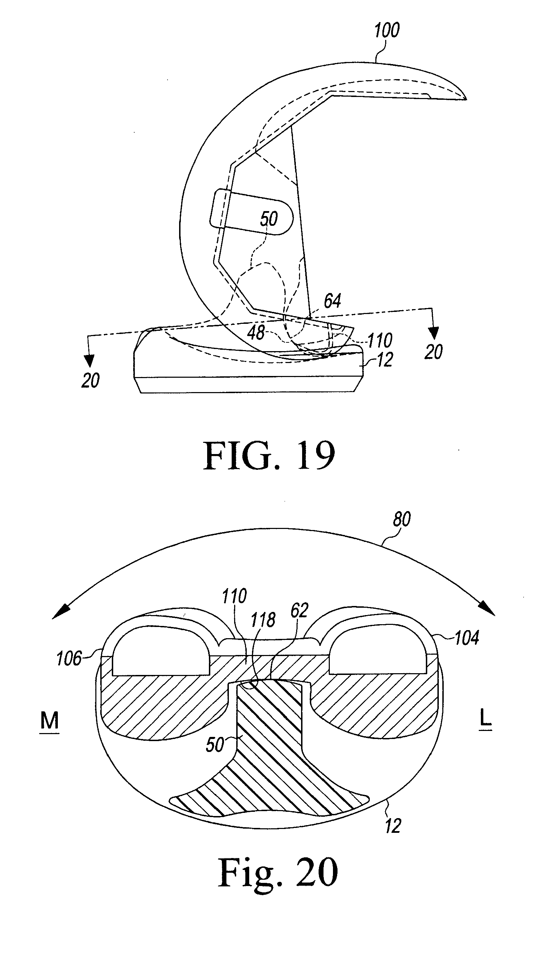

Referring now to FIGS. 19 and 20, when the convex cam surface 118 of the posterior cam 110 is in contact with the concave cam surface 64 of the spine 50 during late flexion, the femoral component 100 may rotate about the spine 50 in a generally medially-laterally direction in the transverse plane as indicated by arrow 82. In such constructions, the convex cam surface 118 of the posterior cam 110 may be substantially planar in the medial-lateral direction. Alternatively, similar to the concave cam surface 64 of the spine 50, the convex cam surface 118 of the posterior cam 110 of the primary femoral component 100 may be curved in the medial-lateral direction. For example, as shown in FIG. 20, the convex cam surface 118 may be concavely curved in the medial-lateral direction. In some constructions, the radius of curvature in the medial-lateral direction of the convex cam surface 118 may be substantially equal to the radius of curvature in the medial-lateral direction of the concave cam surface 64 of the spine 50. Alternatively, the radius of curvature in the medial-lateral direction of the convex cam surface 118 may be greater or slightly less than the radius of curvature in the medial-lateral direction of the concave cam surface 64. As discussed above in relation to early flexion, the amount of rotation between the primary femoral component 100 and the tibial bearing 12 during late flexion may be adjusted based on the radius of curvatures in the medial-lateral direction of the cam surfaces 118, 64.

The posterior cam 210 of the revision femoral component 200 may be substantially planar in the medial-lateral direction. Alternatively, the posterior cam 210 of the revision femoral component 200 may be curved in the medial-lateral direction in a manner similar to the posterior cam 110 of the primary femoral component 100 discussed above.

## Claims

1. An orthopaedic prosthesis assembly comprising:
a tibial bearing (12) configured to be coupled to a tibial tray, the tibial bearing having a platform (16) and a spine (50) extending upwardly from the platform, the spine having a posterior cam surface (60), the posterior cam surface including a concave cam surface (64) and a convex cam surface (62) and
first and second femoral components (100; 200), each of the first and second femoral components being configured to couple separately with the tibial bearing to articulate with the tibial bearing, and including (i) a pair of spaced apart condyles (104, 106; 204, 206) defining an intercondylar notch (108; 208) between them and (ii) a posterior cam (110; 210) positioned in the intercondylar notch, the posterior cam including a posterior cam surface (114; 214),
in which the posterior cam surface of the first femoral component includes a concave cam surface (116) and a convex cam surface (118), the concave cam surface of the posterior cam being configured to contact the convex cam surface of the spine during a first range of flexion and the convex cam surface of the posterior cam being configured to contact the concave cam surface of the spine during a second range of flexion, and
in which the posterior cam surface of the second femoral component is convex,
characterised _in that the posterior cam surface of the second femoral component does not include a concave section so that, as flexion of the knee joint increases, contact between the posterior cam transitions from contact between the convex cam surface of the posterior cam and the convex cam surface of the spine to contact between the convex cam surface of the posterior cam and the concave surface of the spine.

2. The orthopaedic prosthesis assembly of claim 1, in which the concave cam surface (116) of the posterior cam surface (114) of the first femoral component (100) is concavely curved in the sagittal plane and the convex cam surface (118) of the posterior cam surface of the first femoral component is convexly curved in the sagittal plane.

3. The orthopaedic prosthesis assembly of claim 2, in which the concave cam surface (116) and the convex cam surface (118) of the posterior cam surface (114) of the first femoral component (100) are concavely curved in a medial-lateral direction.

4. The orthopaedic prosthesis assembly of claim 3, in which the posterior cam surface (214) of the second femoral component (200) is concavely curved in the medial-lateral direction.

5. The orthopaedic prosthesis assembly of claim 1, in which the posterior cam surfaces (114; 214) of the first and second femoral components (100; 200) are each concavely curved in a medial-lateral direction.

6. The orthopaedic prosthesis assembly of claim 1, in which the first femoral component (100) is a primary femoral component and the second femoral component (200) is a secondary femoral component.

7. The orthopaedic prosthesis assembly of claim 1, in which the convex cam surface (62) of the spine (50) of the tibial bearing (12) is convexly curved in the sagittal plane and the concave cam surface (64) of the spine is concavely curved in the sagittal plane.

8. The orthopaedic prosthesis assembly of claim 7, in which the concave cam surface (64) and the convex cam surface (62) of the spine (50) are convexly curved in the transverse plane.

9. The orthopaedic prosthesis assembly of claim 8, in which the concave cam surface (64) of the spine (50) has a radius of curvature in the transverse plane and the convex cam surface (62) of the spine has a radius of curvature in the transverse plane that is substantially equal to the radius of curvature of the concave cam surface of the spine.

10. The orthopaedic prosthesis assembly of claim 1, in which the convex cam surface (62) of the spine (50) of the tibial bearing (12) is located superiorly relative to the concave cam surface (64) of the spine.

11. The orthopaedic prosthesis assembly of claim 1, in which the degrees of flexion of the first range of flexion are less than the degrees of flexion of the second range of flexion.

12. The orthopaedic prosthesis assembly of claim 1, in which the concave cam surface (64) of the spine (50) of the tibial bearing (12) is defined by a first radius of curvature and the convex cam surface (62) of the spine is defined by a second radius of curvature, the first radius of curvature being different from the second radius of curvature.

13. The orthopaedic prosthesis assembly of claim 12, in which the concave cam surface (116) of the posterior cam surface (114) of the first femoral component (100) is defined by a third radius of curvature and the convex cam surface (118) of the posterior cam surface of the first femoral component is defined by a fourth radius of curvature, the third radius of curvature being different from the fourth radius of curvature.

## Patentansprüche

1. Orthopädische Prothesenanordnung, die umfasst:
ein Schienbeinlager (12), das aufgebaut ist, um mit einer Schienbeinplatte gekoppelt zu werden, wobei das Schienbeinlager eine Plattform (16) und einen Grat (50) hat, der sich von der Plattform nach oben erstreckt, wobei der Grat eine hintere Nockenfläche (60) hat, wobei die hintere Nockenfläche eine konkave Nockenfläche (64) und eine konvexe Nockenfläche (62) hat, und
erste und zweite Oberschenkelkomponenten (100; 200), wobei jede der ersten und zweiten Oberschenkelkomponenten aufgebaut ist, um getrennt mit dem Schienbeinlager zu koppeln, um mit dem Schienbeinlager ein Gelenk zu bilden, und (1) ein Paar beabstandeter Gelenkköpfe (104, 106; 204, 206), die eine interkondyläre Nut (108; 208) zwischen sich definieren, und (ii) eine hintere Nocke (110; 210), die zwischen der interkondylären Nut positioniert ist, umfasst, wobei die hintere Nocke eine hintere Nockenfläche (114; 214) umfasst,
wobei die hintere Nockenfläche der ersten Oberschenkelkomponente eine konkave Nockenfläche (116) und eine konvexe Nockenfläche (118) umfasst, wobei die konkave Nockenfläche der hinteren Nocke aufgebaut ist, um während eines ersten Beugungsbereichs die konvexe Nockenfläche des Grats zu berühren, und die konvexe Nockenfläche der hinteren Nocke aufgebaut ist, um während eines zweiten Beugungsbereichs die konkave Nockenfläche des Grats zu berühren, und
wobei die hintere Nockenfläche der zweiten Oberschenkelkomponente konvex ist,
**dadurch gekennzeichnet, dass** die hintere Nockenfläche der zweiten Oberschenkelkomponente keinen konkaven Abschnitt umfasst, so dass, wenn die Beugung des Kniegelenks zunimmt, der Kontakt zwischen der hinteren Nocke von dem Kontakt zwischen der konvexen Nockenfläche der hinteren Nocke und der konvexen Nockenfläche des Grats in den Kontakt zwischen der konvexen Nockenfläche der hinteren Nocke und der konkaven Fläche des Grats übergeht.

2. Orthopädische Prothesenanordnung nach Anspruch 1, wobei die konkave Nockenfläche (116) der hinteren Nockenfläche (114) der ersten Oberschenkelkomponente (100) in der Sagittalebene konkav gekrümmt ist und die konvexe Nockenfläche (118) der hinteren Nockenfläche der ersten Oberschenkelkomponente in der Sagittalebene konvex gekrümmt ist.

3. Orthopädische Prothesenanordnung nach Anspruch 2, wobei die konkave Nockenfläche (116) und die konvexe Nockenfläche (118) der hinteren Nockenfläche (114) der ersten Oberschenkelkomponente (100) in einer Medial-Lateralrichtung konkav gekrümmt sind.

4. Orthopädische Prothesenanordnung nach Anspruch 3, wobei die hintere Nockenfläche (214) der zweiten Oberschenkelkomponente (200) in der Medial-Lateral-Richtung konkav gekrümmt ist.

5. Orthopädische Prothesenanordnung nach Anspruch 1, wobei die hinteren Nockenflächen (114; 214) der ersten und zweiten Oberschenkelkomponenten (100; 200) jeweils in einer Medial-Lateralrichtung konkav gekrümmt sind.

6. Orthopädische Prothesenanordnung nach Anspruch 1, wobei die erste Oberschenkelkomponente (100) eine primäre Oberschenkelkomponente und die zweite Oberschenkelkomponente (200) eine sekundäre Oberschenkelkomponente ist.

7. Orthopädische Prothesenanordnung nach Anspruch 1, wobei die konvexe Nockenfläche (62) des Grats (50) des Schienbeinlagers (12) in der Sagittalebene konvex gekrümmt ist und die konkave Nockenfläche (64) des Grats in der Sagittalebene konkav gekrümmt ist.

8. Orthopädische Prothesenanordnung nach Anspruch 7, wobei die konkave Nockenfläche (64) und die konvexe Nockenfläche (62) des Grats (50) in der Transversalebene konvex gekrümmt sind.

9. Orthopädische Prothesenanordnung nach Anspruch 8, wobei die konkave Nockenfläche (64) des Grats (50) einen Krümmungsradius in der Transversalebene hat und die konvexe Nockenfläche (62) des Grats einen Krümmungsradius in der Transversalebene hat, der im Wesentlichen gleich dem Krümmungsradius der konkaven Nockenfläche des Grats ist.

10. Orthopädische Prothesenanordnung nach Anspruch 1, wobei die konvexe Nockenfläche (62) des Grats (50) des Schienbeinlagers (12) relativ zu der konkaven Nockenfläche (64) des Grats höher angeordnet ist.

11. Orthopädische Prothesenanordnung nach Anspruch 1, wobei die Beugungsgrade des ersten Beugungsbereichs kleiner als die Beugungsgrade des zweiten Beugungsbereichs sind.

12. Orthopädische Prothesenanordnung nach Anspruch 1, wobei die konkave Nockenfläche (64) des Grats (50) des Schienbeinlagers (12) durch einen ersten Krümmungsradius definiert ist, und die konvexe Nockenfläche (62) des Grats durch einen zweiten Krümmungsradius definiert ist, wobei der erste Krümmungsradius verschieden von dem zweiten Krümmungsradius ist.

13. Orthopädische Prothesenanordnung nach Anspruch 12, wobei die konkave Nockenfläche (116) der hinteren Nockenfläche (114) der ersten Oberschenkelkomponente (100) durch einen dritten Krümmungsradius definiert ist und die konvexe Nockenfläche (118) der hinteren Nockenfläche der ersten Oberschenkelkomponente durch einen vierten Krümmungsradius definiert ist, wobei der dritte Krümmungsradius verschieden von dem vierten Krümmungsradius ist.

## Revendications

1. Ensemble prothèse orthopédique comprenant :
un support tibial (12) configuré pour être couplé à un plateau tibial, le support tibial ayant une plateforme (16) et une arête (50) s'étendant vers le haut à partir de la plateforme, l'arête ayant une surface de came postérieure (60), la surface de came postérieure comprenant une surface de came concave (64) et une surface de came convexe (62) et
des premier et second composants fémoraux (100 ; 200), chacun des premier et second composants fémoraux étant configuré pour se coupler séparément au support tibial pour s'articuler au support tibial, et comprenant (i) une paire de condyles espacés (104, 106 ; 204, 206) définissant une échancrure intercondylienne (108 ; 208) entre eux et (ii) une came postérieure (110 ; 210) positionnée dans l'échancrure intercondylienne, la came postérieure comprenant une surface de came postérieure (114 ; 214),
dans lequel la surface de came postérieure du premier composant fémoral comprend une surface de came concave (116) et une surface de came convexe (118), la surface de came concave de la came postérieure étant configurée pour contacter la surface de came convexe de l'arête sur une première plage de flexion et la surface de came convexe de la came postérieure étant configurée pour contacter la surface de came concave de l'arête sur une seconde plage de flexion, et
dans lequel la surface de came postérieure du second composant fémoral est convexe,
**caractérisé en ce que** la surface de came postérieure du second composant fémoral n'inclut pas de section concave de sorte que, alors que la flexion de l'articulation du genou augmente, le contact entre la came postérieure passe d'un contact entre la surface de came convexe de la came postérieure et la surface de came convexe de l'arête à un contact entre la surface de came convexe de la came postérieure et la surface concave de l'arête.

2. Ensemble prothèse orthopédique selon la revendication 1, dans lequel la surface de came concave (116) de la surface de came postérieure (114) du premier composant fémoral (100) est incurvée de manière concave dans le plan sagittal et la surface de came convexe (118) de la surface de came postérieure du premier composant fémoral est incurvée de manière convexe dans le plan sagittal.

3. Ensemble prothèse orthopédique selon la revendication 2, dans lequel la surface de came concave (116) et la surface de came convexe (118) de la surface de came postérieure (114) du premier composant fémoral (100) sont incurvées de manière concave dans une direction médio-latérale.

4. Ensemble prothèse orthopédique selon la revendication 3, dans lequel la surface de came postérieure (214) du second composant fémoral (200) est incurvée de manière concave dans la direction médio-latérale.

5. Ensemble prothèse orthopédique selon la revendication 1, dans lequel les surfaces de came postérieure (114 ; 214) des premier et second composants fémoraux (100 ; 200) sont chacune incurvées de manière concave dans une direction médio-latérale.

6. Ensemble prothèse orthopédique selon la revendication 1, dans lequel le premier composant fémoral (100) est un composant fémoral primaire et le second composant fémoral (200) est un composant fémoral secondaire.

7. Ensemble prothèse orthopédique selon la revendication 1, dans lequel la surface de came convexe (62) de l'arête (50) du support tibial (12) est incurvée de manière convexe dans le plan sagittal et la surface de came concave (64) de l'arête est incurvée de manière concave dans le plan sagittal.

8. Ensemble prothèse orthopédique selon la revendication 7, dans lequel la surface de came concave (64) et la surface de came convexe (62) de l'arête (50) sont incurvées de manière convexe dans le plan transversal.

9. Ensemble prothèse orthopédique selon la revendication 8, dans lequel la surface de came concave (64) de l'arête (50) a un rayon de courbure dans le plan transversal et la surface de came convexe (62) de l'arête a un rayon de courbure dans le plan transversal qui est sensiblement égal au rayon de courbure de la surface de came concave de l'arête.

10. Ensemble prothèse orthopédique selon la revendication 1, dans lequel la surface de came convexe (62) de l'arête (50) du support tibial (12) est positionnée plus haut relativement à la surface de came concave (64) de l'arête.

11. Ensemble prothèse orthopédique selon la revendication 1, dans lequel les degrés de flexion de la première plage de flexion sont inférieurs aux degrés de flexion de la seconde plage de flexion.

12. Ensemble prothèse orthopédique selon la revendication 1, dans lequel la surface de came concave (64) de l'arête (50) du support tibial (12) est définie par un premier rayon de courbure et la surface de came convexe (62) de l'arête est définie par un deuxième rayon de courbure, le premier rayon de courbure étant différent du deuxième rayon de courbure.

13. Ensemble prothèse orthopédique selon la revendication 12, dans lequel la surface de came concave (116) de la surface de came postérieure (114) du premier composant fémoral (100) est définie par un troisième rayon de courbure et la surface de came convexe (118) de la surface de came postérieure du premier composant fémoral est définie par un quatrième rayon de courbure, le troisième rayon de courbure étant différent du quatrième rayon de courbure.
